# EUROPEAN PATENT APPLICATION

(11) **EP 2 494 992 A1**
(43) Date of publication of application: **05.09.2012**
(21) Application number: 12166176.3
(22) Date of filing: 19.08.2005
(51) Int. Cl.: A61K 45/06, A61K 31/4152, A61K 31/4439

(54) **Combination therapy for treating cyclooxygenase-2 mediated diseases or conditions in patients at risk of thrombotic cardiovascular events**

(30) Priority: 24.08.2004 US 604124 P
(62) Divisional of application: 05858077.0
(71) Applicant: Merck Sharp & Dohme Corp., Rahway, NJ 07065-0907 (US)
(72) Inventor: Thomas, Simon, J., Rahway, NJ New Jersey 07065-0907 (US); Reicin, Alise, S., Rahway, NJ New Jersey 07065-0907 (US); Hunt, Richard, Rahway, NJ New Jersey 07065-0907 (US)
(74) Representative: Böhles, Elena

(57) **Abstract**

The invention encompasses a pharmaceutical composition comprising a therapeutically effective amount of a cyclooxygenase-2 selective inhibitor selected from rofecoxib and etoricoxib or a pharmaceutically acceptable salt thereof, and a therapeutically effective amount of a proton pump inhibitor selected from the group consisting of: omeprazole, lansoprazole, rabeprazole, pantoprazole, and esomeprazole, or a pharmaceutically acceptable salt of any of the aforementioned, in combination with a pharmaceutically acceptable carrier. The invention also encompasses a method for treating a cyclooxygenase-2 mediated disease or condition in a human patient at risk of a thrombotic cardiovascular event, wherein the patient is on aspirin therapy to reduce the risk of the thrombotic cardiovascular event, comprising administering to the patient this pharmaceutical composition.

## Description

### BACKGROUND OF THE INVENTION

Selective inhibitors of cyclooxygenase-2 are a sub-class of the class of drugs known as non-steroidal antiinflammatory drugs (NSAIDs). The NSAIDs are active in reducing the prostaglandin-induced pain and swelling associated with the inflammation process but are also active in affecting other prostaglandin-regulated processes not associated with the inflammation process. Thus, use of high doses of most common NSAIDs can produce severe side effects, including life threatening ulcers, that limit their therapeutic potential. An alternative to NSAIDs is the use of corticosteroids, which have even more drastic side effects, especially when long term therapy is involved.

Previous NSAIDs have been found to prevent the production of prostaglandin by inhibiting enzymes in the human arachidonic acid/prostaglandin pathway including the enzyme cyclooxygenase (COX). The discovery that there are two isoforms of the COX enzyme, the first, COX-1, being involved with physiological functions and the second, COX-2, being induced in inflamed tissue, has given rise to a new approach. While conventional NSAIDs block both forms of the enzyme, the identification of the inducible COX-2 enzyme associated with inflammation has provided a viable target of inhibition which more effectively reduces inflammation and produces fewer and less drastic side effects. Many compounds which have activity as COX-2 inhibitors have been identified, including rofecoxib (VIOXX^{®}), etoricoxib (ARCOXIA™), celecoxib (CELEBREX®) and valdecoxib (BEXTRA™), and much research continues in this area.

Many patients with a chronic cyclooxygenase-2 mediated disease or condition are elderly and thus are at increased risk for thrombotic cardiovascular events, such as stroke, myocardial ischemia, myocardial infarction, angina pectoris, transient ischemic attack (TIA; amaurosis fugax), reversible ischemic neurologic deficits, and any similar thrombotic event in any vascular bed (splanchnic, renal, aortic, peripheral, etc.). Moreover, there is evidence that patients with chronic inflammatory conditions, such as rheumatoid arthritis and systemic lupus erythematosis are at increased risk for thrombotic cardiovascular events. It is desirable that such patients receive appropriate therapy to reduce their risk of such events, such as low-dose aspirin therapy. However, it has been reported that the co-administration of aspirin and a selective COX-2 inhibitor in a rat model resulted in substantially more severe gastric injury than is produced with either agent alone. *See* Fiorucci et al., Gastroenterology, vol. 123, pp. 1598-1606, 2002. In a 12-week endoscopy study conducted in osteoarthritis patients treated with low-dose enteric coated aspirin 81 mg daily, low-dose enteric coated aspirin 81 mg plus VIOXX 25 mg daily, ibuprofen 2400 mg daily, or placebo, there was no difference in the cumulative incidence of endoscopic gastroduodenal ulcers in patients taking low-dose aspirin plus VIOXX 25 mg as compared to those taking ibuprofen 2400 mg daily alone. See prescribing information for VIOXX, Merck & Co., Inc. Thus, the major advantage that COX-2 selective inhibitors have over NSAIDS may be partially offset by the concomitant use of aspirin.

The present invention provides for a combination of a cycloxygenase-2 selective inhibitor selected from rofecoxib and etoricoxib in combination with a proton pump inhibitor for administration to patients on low-dose aspirin therapy. Thus, the invention provides efficacy in treating cyclooxygenase-2 mediated diseases or conditions, effectively inhibits platelets thus reducing the risk of thrombotic cardiovascular events and at the same time reduces the risk of GI ulceration or bleeding relative to the co-administration of a COX-2 inhibitor and low-dose aspirin.

### SUMMARY OF THE INVENTION

The invention encompasses a pharmaceutical composition comprising a therapeutically effective amount of a cyclooxygenase-2 selective inhibitor selected from rofecoxib and etoricoxib or a pharmaceutically acceptable salt thereof, and a therapeutically effective amount of a proton pump inhibitor selected from the group consisting of: omeprazole, lansoprazole, rabeprazole, pantoprazole, and esomeprazole, or a pharmaceutically acceptable salt of any of the aforementioned, in combination with a pharmaceutically acceptable carrier. The invention also encompasses a method for treating a cyclooxygenase-2 mediated disease or condition in a human patient at risk of a thrombotic cardiovascular event, wherein the patient is on aspirin therapy to reduce the risk of the thrombotic cardiovascular event, comprising administering to the patient this pharmaceutical composition.

### DETAILED DESCRIPTION OF THE INVENTION

The inventions encompasses a pharmaceutical composition comprising a therapeutically effective amount of cyclooxygenase-2 selective inhibitor selected from the group consisting of: rofecoxib and etoricoxib or a pharmaceutically acceptable salt thereof, and a therapeutically effective amount of a proton pump inhibitor selected from the group consisting of: omeprazole, lansoprazole, rabeprazole, pantoprazole, and esomeprazole, or a pharmaceutically acceptable salt of any of the aforementioned, in combination with a pharmaceutically acceptable carrier.

An embodiment of the invention encompasses the above pharmaceutical composition wherein the cyclooxygenase-2 selective inhibitor is rofecoxib.

An embodiment of the invention encompasses the above pharmaceutical composition wherein rofecoxib is present in an amount selected from the group consisting of: 12.5 mg, 25 mg and 50 mg.

An embodiment of the invention encompasses the above pharmaceutical composition wherein the cyclooxygenase-2 selective inhibitor is etoricoxib.

An embodiment of the invention encompasses the above pharmaceutical composition wherein etoricoxib is present in an amount selected from the group consisting of: 30 mg, 60 mg, 90 mg and 120 mg.

Another embodiment of the invention encompasses the above pharmaceutical composition wherein the proton pump inhibitor is omeprazole or omeprazole magnesium, each present in an amount selected from the group consisting of: 10 mg, 20 mg and 40 mg.

Another embodiment of the invention encompasses the above pharmaceutical composition wherein the proton pump inhibitor is lansoprazole present in an amount selected from the group consisting of: 15 mg and 30 mg.

Another embodiment of the invention encompasses the above pharmaceutical composition wherein the proton pump inhibitor is rabeprazole sodium present in an amount of 20 mg.

Another embodiment of the invention encompasses the above pharmaceutical composition wherein the proton pump inhibitor is pantoprazole present in an amount selected from the group consisting of: 20 mg and 40 mg.

Another embodiment of the invention encompasses the above pharmaceutical composition wherein the proton pump inhibitor is esomeprazole present in an amount selected from the group consisting of: 20 mg and 40 mg.

Another embodiment of the invention encompasses the above pharmaceutical composition in a capsule form. Another embodiment of the invention encompasses the above pharmaceutical composition in a tablet form.

Another embodiment of the invention encompasses a method for treating a cyclooxygenase-2 mediated disease or condition in a human patient at risk of a thrombotic cardiovascular event, wherein the patient is on aspirin therapy to reduce the risk of the thrombotic cardiovascular event, comprising administering to the patient any of the pharmaceutical compositions described above.

Another embodiment of the invention encompasses this method wherein the thrombotic cardiovascular event is selected from the group consisting of: thrombotic or thromboembolic stroke, myocardial ischemia, myocardial infarction, angina pectoris, transient ischemic attack and reversible ischemic neurologic deficits.

Another embodiment of the invention encompasses this method wherein the patient has had ischemic stroke or transient ischemia of the brain due to fibrin platelet emboli and said patient is on aspirin therapy of about 50 to about 325 mg once daily.

Another embodiment of the invention encompasses this method wherein the patient had a previous myocardial infarction or has unstable angina pectoris and said patient is on aspirin therapy of about 75 to about 325 mg once daily.

Another embodiment of the invention encompasses this method wherein the patient has chronic stable angina pectoris and said patient is on aspirin therapy of about 75 to about 325 mg once daily.

Another embodiment of the invention encompasses this method wherein the patient has peripheral vascular disease and said patient is on aspirin therapy of about 75 to about 325 mg once daily.

Another embodiment of the invention encompasses this method wherein the patient had coronary artery bypass graft surgery and said patient is on aspirin therapy of about 75 to about 325 mg once daily.

Another embodiment of the invention encompasses this method wherein the patient had coronary angioplasty and said patient is on aspirin therapy of about 75 to about 325 mg once daily.

Another embodiment of the invention encompasses this method wherein the patient had carotid endarterectomy and said patient is on aspirin therapy of about 75 to about 325 mg once daily.

Another embodiment of the invention encompasses this method wherein the cyclooxygenase-2 mediated disease or condition is selected from the group consisting of: osteoarthritis, rheumatoid arthritis, chronic and acute pain, including chronic low back pain, primary dysmenorrhea, acute gouty arthritis and ankylosing spondylitis.

Another embodiment of the invention encompasses this method wherein the pharmaceutical composition is administered on a once daily basis.

Another embodiment of the invention encompasses a method for treating a chronic cyclooxygenase-2 mediated disease or condition and reducing the risk of a thrombotic cardiovascular event in a human patient in need of such treatment and at risk of a thrombotic cardiovascular event comprising orally concomitantly or sequentially administering to said patient a cyclooxygenase-2 selective inhibitor selected from rofecoxib and etoricoxib or a pharmaceutically acceptable salt thereof in an amount effective to treat the cyclooxygenase-2 mediated disease or condition, aspirin in an amount effective to reduce the risk of the thrombotic cardiovascular event, and a gastroprotective amount of a proton pump inhibitor selected from the group consisting of: omeprazole, lansoprazole, rabeprazole, pantoprazole, and esomeprazole, or a pharmaceutically acceptable salt of any of the aforementioned.

Another embodiment of the invention encompasses a pharmaceutical composition comprising a therapeutically effective amount of cyclooxygenase-2 selective inhibitor selected from the group consisting of: rofecoxib and etoricoxib or a pharmaceutically acceptable salt thereof, and a therapeutically effective amount of an antithrombotic/anticoagulant/antiplatelet agent selected from the group consisting of: bivalirudin, (Angiomax®); lepirudin, (Refludan®); various heparins; danaparoid, (Orgaran®); various low molecular weight heparins; dalteparin (Fragmin®); enoxaparin (Lovenox®); tinzaparin, (Innohep®); warfarin, (Coumadin®); dicumarol, (Dicoumarol®); anisindione, (Miradone®); argatroban, (Argatroban®); abciximab, (Reopro®); eptifibatide, (Integrilin®); tirofiban, (Aggrastat®); clopidogrel, (Plavix®); ticlopidine, (Ticlid®); and dipyridamole, (Persantine®).

The invention also encompasses a method for treating a chronic cyclooxygenase-2 mediated disease or condition and reducing the risk of a thrombotic cardiovascular event in a human patient in need of such treatment and at risk of a thrombotic cardiovascular event comprising orally concomitantly or sequentially administering to said patient a cyclooxygenase-2 selective inhibitor selected from rofecoxib and etoricoxib or a pharmaceutically acceptable salt thereof in an amount effective to treat the cyclooxygenase-2 mediated disease or condition, and an antithrombotic/anticoagulant/antiplatelet agent selected from the group consisting of: bivalirudin, (Angiomax®); lepirudin, (Refludan®); various heparins; danaparoid, (Orgaran®); various low molecular weight heparins; dalteparin (Fragmin®); enoxaparin (Lovenox®); tinzaparin, (Innohep®); warfarin, (Coumadin®); dicumarol, (Dicoumarol®); anisindione, (Miradone®); argatroban, (Argatroban®); abciximab, (Reopro®); eptifibatide, (Integrilin®); tirofiban, (Aggrastat®); clopidogrel, (Plavix®); ticlopidine, (Ticlid®); and dipyridamole, (Persantine®), in an amount effective to reduce the risk of the cardiovascular event.

Rofecoxib is known in the art and commercially available (VIOXX, Merck & Co., Inc.). Rofecoxib is described as Example 23 in U.S. No. 5,474,995, granted December 12, 1995. Methods for making rofecoxib are described in U.S. No. 5,840,924, granted November 24, 1998.

Etoricoxib is known in the art and commercially available (ARCOXIA, Merck & Co., Inc.). Etoricoxib is described as Example 23 in U.S. No. 5,861,419, granted January 19, 1999. Methods for making etoricoxib are described in U.S. No. 6,040,319, granted March 21, 2000.

The proton pump inhibitors employed in the present invention are commercially available, e.g., omeprazole (PRILOSEC, AstraZeneca), lansoprazole (PREVACID, TAP Pharmaceuticals), rabeprazole (ACIPHEX, Janssen Pharmaceutica), pantoprazole (PROTONIX, Wyeth-Ayerst), and esomeprazole (NEXIUM, AstraZeneca).

The term "treating a chronic cylcooxygenase-2 mediated disease or condition" means treating or preventing any acute or chronic disease or condition that is treated or prevented by inhibiting the cyclooxygenase-2 enzyme. The term includes the relief of pain, fever and inflammation of a variety of conditions including rheumatic fever, symptoms associated with influenza or other viral infections, common cold, low back and neck pain, dysmenorrhea, headache, migraine (acute and prophylactice treatment), toothache, sprains and strains, myositis, neuralgia, synovitis, arthritis, including rheumatoid arthritis, degenerative joint diseases (osteoarthritis), gout and ankylosing spondylitis, acute, subacute and chronic musculoskeletal pain syndromes such as bursitis, bums, injuries, and pain following surgical and dental procedures as well as the preemptive treatment of surgical pain. In addition, the term includes the inhibition cellular neoplastic transformations and metastic tumor growth and hence the treatment of cancer. The term also includes the treatment of endometriosis and Parkinson's disease as well as the treatment of cyclooxygenase-mediated proliferative disorders such as may occur in diabetic retinopathy and tumor angiogenesis. The term "treating" encompasses not only treating a patient to relieve the patient of the signs and symptoms of the disease or condition but also prophylactically treating an asymptomatic patient to prevent the onset or progression of the disease or condition.

A "thrombotic cardiovascular event" is defined as any sudden event of a type known to be caused by platelet aggregation, thrombosis, and subsequent ischemic clinical events, including thrombotic or thromboembolic stroke, myocardial ischemia, myocardial infarction, angina pectoris, transient ischemic attack (TIA; amaurosis fugax), reversible ischemic neurologic deficits, and any similar thrombotic event in any vascular bed (splanchnic, renal, aortic, peripheral, etc.).

A "patient at risk of a thrombotic cardiovascular event" can readily be diagnosed by one having ordinary skill in the art. For example, such a patient includes those who have had ischemic stroke or transient ischemia of the brain due to fibrin platelet emboli, those with a previous myocardial infarction or unstable angina pectoris, and those with chronic stable angina pectoris. Risk factors for a thrombotic cardiovascular event include hypertension, hypercholesterolemia, diabetes mellitus, chronic renal impairment, smoking, and any prior personal or family history of such an event. A patient with one or more of the aforementioned risk factors is included within the scope of the present invention. The patient at risk of a thrombotic cardiovascular event according to the invention would be on aspirin therapy to reduce the risk of the cardiovascular event. The term "aspirin therapy to reduce the risk of the cardiovascular event" is well understood in the art as low-dose aspirin is indicated for these conditions. Administration of the drug combination to the patient includes both self-administration and administration to the patient by another person.

The terms "therapeutically effective amount" and "gastroprotective amount" is intended to mean that amount of a drug or pharmaceutical agent that will elicit the biological or medical response of a tissue, a system, animal or human that is being sought by a researcher, veterinarian, medical doctor or other clinician. The term also encompasses the amount of a pharmaceutical drug that will prevent or reduce the risk of occurrence of the biological or medical event that is sought to be prevented in a tissue, a system, animal or human by a researcher, veterinarian, medical doctor or other clinician. Conventional dosage amounts may be employed with the instant invention. For example, etoricoxib may be administered at 30 mg, 60 mg, 90 mg or 120 mg to treat or prevent a cycloxygenase-2 mediated disease. Esomeprazole may be employed at 20 mg or 40 mg for its gastroprotective effects.

For vascular indications, aspirin is typically administered at a dose of about 30 mg to about 1 g once daily, preferably at a dose of about 80 mg to about 650 mg.

The term "concomitantly administering" means administering the agents substantially concurrently. The term "concomitantly administering" encompasses not only administering the two agents in a single pharmaceutical dosage form but also the administration of each active agent in its own separate pharmaceutical dosage formulation. Where separate dosage formulations are used, the agents can be administered at essentially the same time, i.e., concurrently.

The term "sequentially administering" means administering the agents at separately staggered times. Thus, agents can be sequentially administered such that the beneficial pharmaceutical effects of the active agents are realized by the patient at substantially the same time. Thus, for example, if agents are administered on a once a day basis, the interval of separation between sequential administration of the agents can be up to twelve hours apart.

The pharmaceutical compositions of the present invention comprise rofecoxib or etoricoxib and a proton pump inhibitor as active ingredients, or pharmaceutically acceptable salts thereof, and may also contain a pharmaceutically acceptable carrier and optionally other therapeutic ingredients. The term "pharmaceutically acceptable salts" include salts prepared from bases that result in non-toxic pharmaceutically acceptable salts, including inorganic bases and organic bases. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

When compounds of the present invention are basic, salts may be prepared from acids that result in pharmaceutically acceptable salts, including inorganic and organic acids. Such acids include acetic, adipic, aspartic, 1,5-naphthalenedisulfonic, benzenesulfonic, benzoic, camphorsulfonic, citric, 1,2-ethanedisulfonic, ethanesulfonic, ethylenediaminetetraacetic, fumaric, glucoheptonic, gluconic, glutamic, hydriodic, hydrobromic, hydrochloric, isethionic, lactic, maleic, malic, mandelic, methanesulfonic, mucic, 2-naphthalenesulfonic, nitric, oxalic, pamoic, pantothenic, phosphoric, pivalic, propionic, salicylic, stearic, succinic, sulfuric, tartaric, p-toluenesulfonic acid, undecanoic, 10-undecenoic, and the like.

The term "cyclooxygenase-2 selective inhibitors" means compounds that are inhibitors of cyclooxygenase-2 enzyme and are thereby useful in the treatment of cyclooxygenase-2 mediated diseases as enumerated above. This activity is illustrated by their ability to selectively inhibit cyclooxygenase-2 over cyclooxygenase-1. Accordingly, in one assay, the ability of the compounds of this invention to treat cyclooxygenase mediated diseases can be demonstrated by measuring the amount of prostaglandin E₂ (PGE₂) synthesized in the presence of arachidonic acid, cyclooxygenase-1 or cyclooxygenase-2 and a test compound. The IC₅₀ values represent the concentration of inhibitor required to return PGE₂ synthesis to 50% of that obtained as compared to the uninhibited control. The terms "cyclooxyegnase-2 selective inhibitor" includes compounds in any form, including salts, hydrates and polymorphs.

The combination of this invention will be useful for the relief of pain, fever and inflammation of a variety of conditions including rheumatic fever, symptoms associated with influenza or other viral infections, common cold, low back and neck pain, dysmenorrhea, headache, migraine (acute and prophylactice treatment), toothache, sprains and strains, myositis, neuralgia, synovitis, arthritis, including rheumatoid arthritis, degenerative joint diseases (osteoarthritis), gout and ankylosing spondylitis, acute, subacute and chronic musculoskeletal pain syndromes such as bursitis, bums, injuries, and pain following surgical and dental procedures as well as the preemptive treatment of surgical pain. In addition, the combination of this invention may inhibit cellular neoplastic transformations and metastic tumor growth and hence can be used in the treatment of cancer. The combination of this invention may also be useful for the treatment or prevention of endometriosis and Parkinson's disease.

The combination of this invention will also inhibit prostanoid-induced smooth muscle contraction by preventing the synthesis of contractile prostanoids and hence may be of use in the treatment of dysmenorrhea, premature labor and asthma.

The present invention provides for a combination of a cycloxygenase-2 selective inhibitor, selected from rofecoxib and etoricoxib, in combination with a proton pump inhibitor for administration to patients on low-dose aspirin therapy. Thus, the invention provides efficacy in treating cyclooxygenase-2 mediated diseases or conditions via administration of the cyclooxygenase-2 inhibitor, effectively inhibits platelets thus reducing the risk of thrombotic cardiovascular events via administration of low-dose aspirin and at the same time reduces the risk of GI ulceration or bleeding relative to the co-administration of a COX-2 inhibitor and low-dose aspirin via administration of the proton pump inhibitor.

The present invention will prove particularly useful in patients with peptic ulcers, gastritis, regional enteritis, ulcerative colitis, diverticulitis or with a recurrent history of gastrointestinal lesions; GI bleeding, coagulation disorders including anemia such as hypoprothrombinemia, haemophilia or other bleeding problems (including those relating to reduced or impaired platelet function); and those prior to surgery or taking anticoagulants.

In further aspects, the invention encompasses pharmaceutical compositions for treating cyclooxygenase-2 mediated diseases as defined above comprising a pharmaceutical composition comprising etoricoxib or rofecoxib and a proton pump inhibitor and one or more ingredients such as another pain reliever including acetominophen or phenacetin; opioid analgesics, such as codeine, fentanyl, hydromorphone, levorphanol, meperidine, methadone, morphine, oxycodone, oxymorphine, propoxyphene, buprenorphine, butorphanol, dezocine, nalbuphine and pentazocine; a potentiator including caffeine; an H2-antagonist; aluminum or magnesium hydroxide; simethicone; a decongestant including phenylephrine, phenylpropanolamine, pseudophedrine, oxymetazoline, ephinephrine, naphazoline, xylometazoline, propylhexedrine, or levo-desoxyephedrine; an antitussive including codeine, hydrocodone, caramiphen, carbetapentane, or dextramethorphan; a diuretic; and a sedating or non-sedating antihistamine. For the treatment or prevention of migraine, the invention also encompasses co-administration with a 5-HT agonist such as rizatriptan, sumatriptan, zolmitriptan and naratriptan. In addition the invention encompasses a method of treating cyclooxygenase mediated diseases comprising: administration to a patient in need of such treatment a pharmaceutical composition of the present invention, optionally co-administered with one or more of such ingredients as listed immediately above.

The invention includes the treatment of warm-blooded animals such as mice, rats, horses, cattle sheep, dogs, cats, etc., the compound of the invention is effective in the treatment of humans.

The pharmaceutical compositions containing the active ingredients may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the technique described in the U.S. Patent 4,256,108; 4,166,452; and 4,265,874 to form osmotic therapeutic tablets for control release.

An enteric coating may be employed with the pharmaceutical compositions of the present invention to protect the acid labile proton pump inhibitors. Techniques for formulating such drugs with an enteric coating are well known in the art.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredients is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethyl-cellulose, methylcellulose, hydroxypropylmethy-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethylene-oxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose, saccharin or aspartame.

Liquid formulations include the use of self-emulsyfying drug delivery systems and NanoCrystal® technology. Cyclodextrin inclusion complexes can also be utilized.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavoring and coloring agents.

Conventional dosage amount of the active ingredients of compounds of the present invention may be employed in the pharmaceutical compositions of the invention. It will be understood, however, that the specific dose level for any particular patient will depend upon a variety of factors including the age, body weight, general health, sex, diet, time of administration, route of administration, rate of excretion, drug combination and the severity of the particular disease undergoing therapy.

### Gastic Erosion Model

The gastric protective effects of the combination of the present invention co-administered with aspirin may be evaluated in the following assay.

Male Wistar rats (200 - 250 g) are fasted for 16 - 18 h prior to use for experiment. Aspririn, rofecoxib or etoricoxib in combination with aspirin (dosed separately), or proton pump inhibitor in combination with rofecoxib or etoricoxib and aspirin (dosed separately) are given on the morning of the experiment at a dosing volume of 1 ml/kg in 0.5% methocel. Three hr later, the animals are euthanized by CO₂ inhalation and the stomach removed, rinsed in saline and prepared for imaging processing. Microscopic pictures of the stomach are taken using a digital camera and gastric erosions are measured using an imaging software by an observer unaware of the treatment groups. The length of gastric erosions is measured in mm and the total length of all erosions from each stomach is obtained and used as a gastric damage score.

This model is also described in S. Fiorucci, et al., Gastroenterology, vol. 123, pp. 1598-1606, 2002 and M. Souza, et al., Am. J. Physiol. Gastrointest. Liver Physiol., vol. 285, pp. G54-G61, 2003.

## Claims

1. A pharmaceutical composition comprising a therapeutically effective amount of cyclooxygenase-2 selective inhibitor selected from the group consisting of: rofecoxib and etoricoxib or a pharmaceutically acceptable salt thereof, and a therapeutically effective amount of a proton pump inhibitor selected from the group consisting of: omeprazole, lansoprazole, rabeprazole, pantoprazole, and esomeprazole, or a pharmaceutically acceptable salt of any of the aforementioned, in combination with a pharmaceutically acceptable carrier.

2. The pharmaceutical composition according to Claim 1 wherein the cyclooxygenase-2 selective inhibitor is rofecoxib.

3. The pharmaceutical composition according to Claim 2 wherein rofecoxib is present in an amount selected from the group consisting of: 12.5 mg, 25 mg and 50 mg.

4. The pharmaceutical composition according to Claim 1 wherein the cyclooxygenase-2 selective inhibitor is etoricoxib.

5. The pharmaceutical composition according to Claim 4 wherein etoricoxib is present in an amount selected from the group consisting of: 30 mg, 60 mg, 90 mg and 120 mg.

6. The pharmaceutical composition according to any one of Claims 1-5 wherein the proton pump inhibitor is omeprazole or omeprazole magnesium, each present in an amount selected from the group consisting of: 10 mg, 20 mg and 40 mg.

7. The pharmaceutical composition according to any one of Claims 1-5 wherein the proton pump inhibitor is lansoprazole present in an amount selected from the group consisting of: 15 mg and 30 mg.

8. The pharmaceutical composition according to any one of Claims 1-5 wherein the proton pump inhibitor is rabeprazole sodium present in an amount of 20 mg.

9. The pharmaceutical composition according to any one of Claims 1-5 wherein the proton pump inhibitor is pantoprazole present in an amount selected from the group consisting of: 20 mg and 40 mg.

10. The pharmaceutical composition according to any one of Claims 1-5 wherein the proton pump inhibitor is esomeprazole present in an amount selected from the group consisting of: 20 mg and 40 mg.

11. A combination comprising a cyclooxygenase-2 selective inhibitor selected from the group consisting of: rofecoxib and etoricoxib or a pharmaceutically acceptable salt thereof, and a proton pump inhibitor selected from the group consisting of: omeprazole, lansoprazole, rabeprazole, pantoprazole, and esomeprazole, or a pharmaceutically acceptable salt thereof.

12. Use of a pharmaceutical composition according to any one of Claims 1 to 10 or a combination according to Claim 11 for the manufacture of a medicament for treating a cyclooxygenase-2 mediated disease or condition in a human patient at risk of a thrombotic cardiovascular event, wherein the patient is on aspirin therapy to reduce the risk of the thrombotic cardiovascular event.

13. The use according to Claim 12 wherein the thrombotic cardiovascular event is selected from the group consisting of: thrombotic or thromboembolic stroke, myocardial ischemia, myocardial infarction, angina pectoris, transient ischemic attack and reversible ischemic neurologic deficits.

14. The use according to Claim 12 wherein the patient has had ischemic stroke or transient ischemia of the brain due to fibrin platelet emboli and said patient is on aspirin therapy of about 50 to about 325 mg once daily.

15. The use according to Claim 12 wherein the patient had a previous myocardial infarction or has unstable angina pectoris and said patient is on aspirin therapy of about 75 to about 325 mg once daily.

16. The use according to Claim 12 wherein the patient has chronic stable angina pectoris and said patient is on aspirin therapy of about 75 to about 325 mg once daily.

17. The use according to Claim 12 wherein the cyclooxygenase-2 mediated disease or condition is selected from the group consisting of: osteoarthritis, rheumatoid arthritis, chronic and acute pain, primary dysmenorrhea, acute gouty arthritis and ankylosing spondylitis.

18. Use of a cyclooxygenase-2 selective inhibitor selected from rofecoxib and etoricoxib or a pharmaceutically acceptable salt thereof in an amount effective to treat the cyclooxygenase-2 mediated disease or condition, aspirin in an amount effective to reduce the risk of the thrombotic cardiovascular event, and a gastroprotective amount of a proton pump inhibitor selected from the group consisting of: omeprazole, lansoprazole, rabeprazole, pantoprazole, and esomeprazole, or a pharmaceutically acceptable salt of any of the aforementioned, for the manufacture of a medicament for treating a chronic cyclooxygenase-2 mediated disease or condition and reducing the risk of a thrombotic cardiovascular event in a human patient in need of such treatment and at risk of a thrombotic cardiovascular event.

19. The combination according to Claim11 which further comprises aspirin.
